(19) Europäisches Patentamt
European Patent Office
Office européen des brevets

(11) **EP 3 502 666 B1**

(12) **FASCICULE DE BREVET EUROPEEN**

(45) Date de publication et mention de la délivrance du brevet:
**25.05.2022 Bulletin 2022/21**

(21) Numéro de dépôt: **18214247.1**

(22) Date de dépôt: **19.12.2018**

(51) Classification Internationale des Brevets (IPC):
***G01N 21/47*** *(2006.01)*

(52) Classification Coopérative des Brevets (CPC):
**G01N 21/474;** G01N 2021/4745; G01N 2201/0626

(54) **DISPOSITIF DE MESURE D'UN RAYONNEMENT RÉTRODIFFUSÉ PAR UN ÉCHANTILLON ET PROCÉDÉ DE MESURE UTILISANT UN TEL DISPOSITIF**

MESSVORRICHTUNG EINER RÜCKSTREUSTRAHLUNG VON EINER PROBE, UND MESSVERFAHREN, DAS EINE SOLCHE VORRICHTUNG VERWENDET

DEVICE FOR MEASURING BACKSCATTERED RADIATION BY A SAMPLE AND MEASURING METHOD USING SUCH A DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.12.2017 FR 1763097**

(43) Date de publication de la demande:
**26.06.2019 Bulletin 2019/26**

(73) Titulaire: **Commissariat à l'Énergie Atomique et aux Énergies Alternatives 75015 Paris cedex (FR)**

(72) Inventeurs:
• **PETITDIDIER, Nils 38100 Grenoble (FR)**
• **BENAHMED, Selimen 38000 Grenoble (FR)**
• **KOENIG, Anne 38410 Saint Martin d'Uriage (FR)**

(74) Mandataire: **INNOV-GROUP 310, avenue Berthelot 69372 Lyon Cedex 08 (FR)**

(56) Documents cités:
**EP-A1- 2 743 743        EP-A1- 3 236 241**

WO-A1-96/08201        WO-A1-2016/181077
US-A1- 2016 235 303

• **PETITDIDIER NILS ET AL: "Development of a wearable CMOS-based contact imaging system for real-time skin condition diagnosis", PROGRESS IN BIOMEDICAL OPTICS AND IMAGING, SPIE - INTERNATIONAL SOCIETY FOR OPTICAL ENGINEERING, BELLINGHAM, WA, US, vol. 10412, 28 juillet 2017 (2017-07-28), pages 104120F-104120F, XP060093120, ISSN: 1605-7422, DOI: 10.1117/12.2285998 ISBN: 978-1-5106-0027-0**
• **ANNE KOENIG ET AL: "Diffuse reflectance spectroscopy: a clinical study of tuberculin skin tests reading", PROCEEDINGS OF SPIE, vol. 8592, 21 février 2013 (2013-02-21), pages 85920S-85920S-8, XP055090850, ISSN: 0277-786X, DOI: 10.1117/12.2002314**
• **Brandon S Nichols ET AL: "A Quantitative Diffuse Reflectance Imaging (QDRI) System for Comprehensive Surveillance of the Morphological Landscape in Breast Tumor Margins", PloS one, 15 juin 2015 (2015-06-15), page e0127525, XP055337212, United States DOI: 10.1371/journal.pone.0127525 Extrait de l'Internet: URL:http://journals.plos.org/plosone/article/file?id=10.1371/journal.pone.0127525&type=printable [extrait le 2017-01-20]**

## Description

## DOMAINE TECHNIQUE

La spectrométrie de réflectance diffuse, souvent désignée par l'acronyme anglais DRS (Diffuse Reflectance Spectroscopy), consiste à exploiter la lumière rétrodiffusée par un objet diffusant soumis à un éclairement, généralement ponctuel. Cette technique s'avère performante pour caractériser des propriétés optiques d'objets, en particulier les propriétés de diffusion ou d'absorption.

Mise en œuvre sur la peau, cette technique permet par exemple de caractériser la peau, comme décrit dans le document EP2762064. Les auteurs de ce document décrivent une sonde de mesure destinée à être appliquée contre la peau. Cette sonde comporte une fibre optique centrale, dite fibre d'excitation, destinée à diriger un faisceau lumineux vers un échantillon de peau. Des fibres optiques, disposées autour de la fibre centrale, dites fibres de détection, recueillent un signal optique rétrodiffusé par la peau. Des moyens d'analyse spectrale du signal optique ainsi collecté, couplés à des algorithmes de calcul, permettent d'estimer des paramètres du derme, en particulier la concentration de certains chromophores, par exemple l'oxyhémoglobine ou la déoxyhémoglobine, mais aussi des paramètres gouvernant le parcours des photons dans le derme, notamment le coefficient de diffusion réduit $\mu_s'$ ainsi que le coefficient d'absorption $\mu_a$. Les applications sont nombreuses, et peuvent par exemple permettre de détecter et de quantifier des concentrations d'éléments dans la peau, ou d'accéder à une information relative à l'oxymétrie des vaisseaux sanguins traversant la peau. Des applications sont décrites dans EP 2762064, ainsi que dans la publication Haahr R.,"An Electronic Patch for Wearable Health Monitoring by Reflectance Pulse Oxymetry", IEEE Transactions on biomedical circuits and systems, 2011, ou encore dans le brevet US805532.

Selon les principes de la diffusion de la lumière dans un milieu diffusant, la profondeur moyenne de pénétration des photons dans un objet diffusant augmente avec l'écartement entre la fibre d'excitation et la fibre de détection. Autrement dit, plus la distance entre la fibre d'excitation et la fibre de détection augmente, plus le résultat est représentatif des propriétés optiques des couches profondes du milieu examiné. A l'inverse, plus cette distance diminue, plus le résultat est représentatif des couches superficielles du milieu diffusant.

Or, afin d'effectuer une mesure suffisamment sensible, chaque fibre a un diamètre égal à quelques centaines de microns, typiquement 500 $\mu$m pour la fibre d'excitation et 150 $\mu$m pour chaque fibre de détection. Par ailleurs, du fait de contraintes de fabrication, il est difficilement envisageable de réduire significativement la distance entre ces fibres. Il en résulte que l'écartement minimal entre chaque fibre ne peut pas être inférieur à une valeur seuil, généralement de l'ordre de quelques centaines de $\mu$m, généralement au-delà de 300 $\mu$m.

De ce fait, alors que le dispositif décrit dans EP2762064 est adapté à la caractérisation du derme, il n'est pas apte à caractériser l'épiderme, ce dernier s'étendant, selon les individus et les zones corporelles, sur les 100 à 300 premiers microns de la peau.

Le document WO 2016/181077 A1 décrit un dispositif comprenant une source de lumière d'excitation, illuminant un objet, notamment un tissu biologique et induisant une émission d'une lumière d'émission par l'objet examiné. Selon une variante, un faisceau lumineux d'excitation émis par la source de lumière est transmis par un faisceau de fibres optiques d'excitation. La lumière d'émission est par exemple une lumière de fluorescence et/ou de rétrodiffusion. Le dispositif permet d'obtenir une image du tissu biologique examiné, à partir de la lumière d'émission. Le dispositif comporte également un capteur de télémétrie, émettant un faisceau lumineux de télémétrie vers l'objet. Un élément projecteur permet de projeter le faisceau d'excitation et le faisceau de télémétrie directement sur l'objet. Le capteur de télémétrie permet d'estimer une distance entre l'élément projecteur et l'objet. La mesure de cette distance permet de moduler une intensité de la lumière d'excitation émise par l'objet. Lorsque l'objet est un tissu biologique, cela permet d'une part de respecter des seuils d'illumination de tissus biologiques, et, d'autre part, de maintenir une illumination d'intensité constante. L'élément projecteur peut notamment être inclus à l'extrémité d'un laparoscope ou d'un endoscope.

Le document US 2016/235303 A1 décrit décrit un dispositif de mesure de spectrométrie de réflectance diffuse (NIRS), comportant une source de lumière, un spectromètre ainsi que des fibres optiques souples. Le dispositif de NIRS comporte des fibres optiques, destinées à être insérées dans un cathéter, pour la caractérisation de tissus cardiaques.

Le document EP3054281 décrit un dispositif permettant à remédier à ce problème, en disposant un système optique entre des fibres optiques et un objet à caractériser, par exemple la peau d'un individu. Selon ce dispositif, les fibres optiques sont disposées à distance de l'objet à caractériser, et un système optique, de grossissement supérieur à 1, est disposé entre les fibres et l'objet à caractériser. Le système optique assure un couplage optique des fibres optiqus avec la surface de l'objet caractérisé. Les performances d'un tel dispositif sont bonnes, mais son encombrement ne lui permet d'être porté de façon continue par une personne. Ce dispositif convient davantage à des examens effectués de manière ponctuelle, qu'à un suivi en continu à l'aide d'un dispositif portable. De plus, le coût d'un tel dispositif est relativement élevé. L'invention est une alternative au dispositif décrit EP3054281. Elle permet de caractériser un objet diffusant, notamment les propriétés optiques de la couche superficielle d'un échantillon, très compact et de conception simple.

**EXPOSE DE L'INVENTION**

Un premier objet de l'invention est un dispositif de mesure d'un rayonnement rétrodiffusé par un échantillon selon la revendication 1.

Le dispositif peut comporter une ou plusieurs caractéristiques suivantes, prises isolément ou en combinaison :

- la source de lumière est disposée à la périphérie du faisceau de fibres optique, entre le capteur d'image et l'échantillon ;
- la source de lumière affleure la surface distale ;
- l'axe d'extension est perpendiculaire au plan de détection ;

    - les fibres optiques du faisceau de fibres optiques :
    - sont orientées parallèlement à l'axe d'extension ;

- et/ou forment un faisceau divergent ou convergent selon l'axe d'extension ;
- le dispositif comporte plusieurs sources de lumière, chaque source de lumière étant disposée à la périphérie du faisceau de fibres optiques ;
- au moins deux sources de lumière émettent des faisceaux lumineux respectivement selon des bandes spectrales différentes ;
- au moins une source de lumière est telle que l'axe d'incidence du faisceau lumineux produit par cette dernière est sécant par de l'axe d'extension du faisceau de fibres optiques. L'axe d'incidence et l'axe d'extension peuvent former un angle compris entre 10° et 70° ou 80° ;
- au moins une source de lumière est :

    - une diode électroluminescente ;
    - ou une diode laser ;
    - ou une extrémité d'une fibre optique, dite fibre optique d'illumination, la fibre optique d'illumination étant couplée à une source lumineuse.

- Le capteur d'image et chaque source de lumière sont maintenus solidaires les uns des autres par un support de maintien ;
- le capteur d'image comporte une matrice de pixels, de telle sorte que la distance, dans le plan de détection, entre au moins une source de lumière et la matrice de pixels est inférieure à 1 mm ou inférieure à 500 μm ; Il s'agit de la distance, parallèlement au plan de détection, entre le faisceau incident produit par la source de lumière et la matrice de pixels.

Un deuxième objet de l'invention est un procédé d'estimation d'une propriété optique de diffusion et/ou d'absorption d'un échantillon, à l'aide d'un dispositif selon le premier objet de l'invention, le procédé comportant les étapes suivantes :

a) illumination de l'échantillon à l'aide de la source de lumière du dispositif, de façon à former, sur une surface de l'échantillon, une zone élémentaire d'illumination ;
b) formation, à l'aide du capteur d'image, d'une image représentant un rayonnement rétrodiffusé par l'échantillon sous l'effet de l'illumination, le rayonnement rétrodiffusé émanant de la surface de l'échantillon, à l'extérieur de la zone élémentaire d'illumination, à plusieurs distances, dites distances de rétrodiffusion, de la zone élémentaire d'illumination ;
c) détermination d'une propriété optique de diffusion et/ou d'absorption en fonction d'une intensité du rayonnement rétrodiffusé, à différentes distances de rétrodiffusion, au moins une distance de rétrodiffusion étant inférieure à 1 mm ou à 500 μm.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

**FIGURES**

Les figures 1A et 1B représentent un dispositif selon une configuration connue de l'art antérieur, dite configuration "à distance".
La figure 1C schématise un dispositif selon une configuration au contact, simple à concevoir.

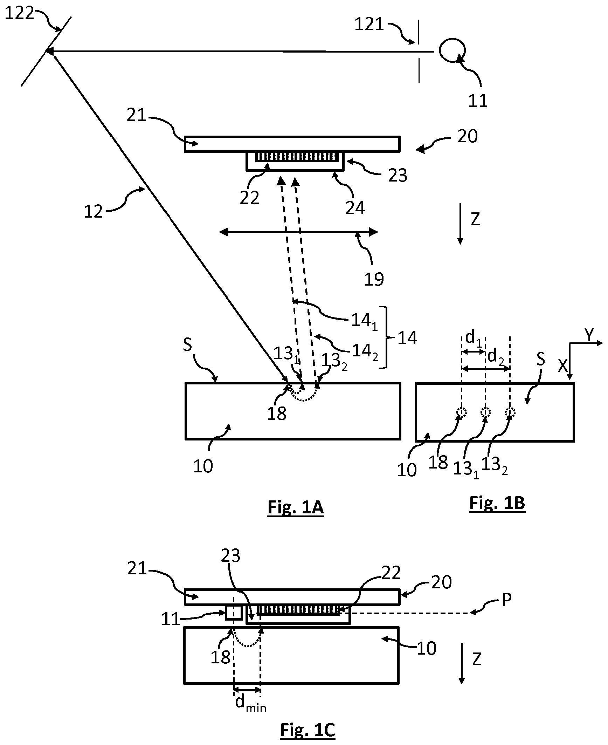

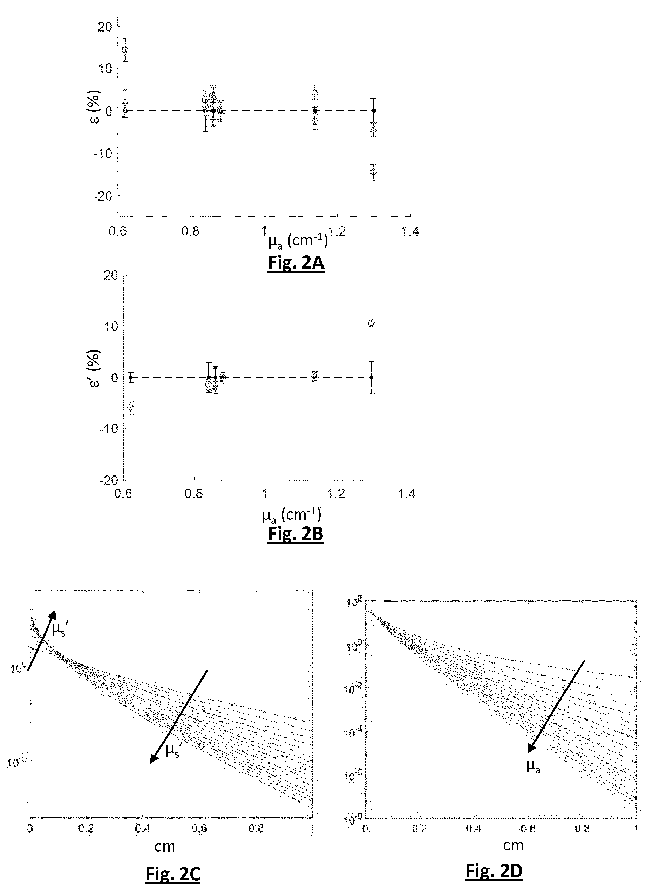

Les figures 2A et 2B rassemblent des résultats de mesures expérimentales réalisées sur des échantillons de calibration, dont les propriétés optiques sont connues, à l'aide de dispositifs selon les configurations présentées en lien avec les figures 1A et 1C. La figure 2A représente une erreur relative de quantification d'un coefficient d'absorption, en fonction du coefficient d'absorption de l'échantillon de calibration. La figure 2B représente une erreur relative de quantification d'un coefficient de diffusion réduit en fonction du coefficient d'absorption de l'échantillon de calibration. La figure 2C représente une évolution de la réflectance d'un tissu en fonction de la distance de rétrodiffusion, pour différentes valeurs du coefficient de diffusion réduit. La figure 2D représente une évolution de la réflectance d'un tissu en fonction de la distance de rétrodiffusion, pour différentes valeurs du coefficient d'absorption.
Les figures 3A à 3G représentent différents modes de réalisation d'un dispositif selon l'invention.
La figure 3H illustre les principales étapes d'un procédé de détermination de propriétés optiques de l'invention en utilisant un dispositif tel que représenté sur les figures 3A à 3C.

Les figures 4A et 4B rassemblent des résultats de mesures expérimentales réalisées sur des échantillons de calibration, dont les propriétés optiques sont connues, à l'aide de dispositifs selon les configurations présentées en lien avec les figures 1A, 1C et 3A. La figure 4A représente une erreur relative de quantification d'un coefficient d'absorption, en fonction du coefficient d'absorption de l'échantillon de calibration. La figure 4B représente une erreur relative de quantification d'un coefficient de diffusion réduit en fonction du coefficient d'absorption de l'échantillon de calibration.

## EXPOSE DE MODES DE REALISATION PARTICULIERS

Par propriété optique de diffusion ou d'absorption, il est entendu un coefficient d'absorption, ou un coefficient de diffusion, ou un coefficient de diffusion réduit, ces grandeurs étant connues de l'homme du métier, et décrites dans la demande EP3054281. A partir des propriétés d'absorption, on peut déterminer une concentration d'un composant de l'échantillon. Par exemple, lorsque l'échantillon est la peau d'un individu ou d'un animal, on peut déterminer une concentration de différents composants de la peau, ainsi que de l'oxyhémoglobine ou de la désoxyhémoglobine du sang circulant dans la peau. Par exemple, si on considère le coefficient d'absorption $\mu_a$, on peut utiliser une expression telle que :

$$\mu_a = f(\textstyle\sum_i \varepsilon_i \, c_i)$$

où

- $c_i$ est une concentration de composants i dans la peau (en mol.L$^{-1}$);
- $\varepsilon_i$ est un coefficient d'extinction de chaque composant i (en L.mol$^{-1}$.cm$^{-1}$)
- f est une fonction reliant $c_i$ et $\varepsilon_i$ à $\mu_a$

Le terme un est à comprendre comme signifiant "au moins un".

On a représenté, sur la figure 1A, une configuration de l'art antérieur, dite configuration "à distance", selon laquelle une source de lumière 11 émet un faisceau d'illumination 12, dans une bande spectrale $\Delta\lambda$, de façon à illuminer un échantillon 10 à caractériser. L'échantillon s'étend selon une surface S. Le faisceau d'illumination 12 passe à travers un diaphragme 121, puis est réfléchi vers l'échantillon 10 par un élément réfléchissant 122. Il parvient sur la surface de l'échantillon selon une incidence oblique. Le faisceau d'illumination 12 forme, à la surface de l'échantillon, une trace lumineuse, dite zone élémentaire d'illumination 18. La zone élémentaire d'illumination correspond à une intersection entre le faisceau lumineux incident 12 et la surface S de l'échantillon. Dans l'exemple représenté, elle est ponctuelle.

Sous l'effet de l'illumination, une partie de la lumière provenant du faisceau d'illumination 12 diffuse dans l'échantillon, et est rétrodiffusée par ce dernier de façon à former un rayonnement rétrodiffusé 14 émanant de la surface S de l'échantillon. Ce dernier se propage vers un système optique 19, optiquement couplé à un capteur d'image 20. La surface de l'échantillon s'étend alors dans un plan focal objet du système optique 19. Ainsi, le système optique 19 transporte l'image du rayonnement de rétrodiffusion 14, émanant de la surface de l'échantillon S, jusqu'au capteur d'image 20. Un tel dispositif est une application de la demande de brevet EP3054281 citée dans l'art antérieur.

Le capteur d'image 20 peut notamment être un capteur de type CMOS ou CCD. Il comporte un support 21, comportant par exemple un circuit de lecture, sur lequel est disposée une matrice de pixels 22, confinée dans une enceinte de protection 23. La matrice de pixels 22 s'étend selon un plan de détection P. L'enceinte de protection 23 comporte une fenêtre transparente 24, s'étendant parallèlement à la matrice de pixels 22. Par transparent, on entend dans la bande spectrale $\Delta\lambda$. Dans l'enceinte de protection 23 s'étendent de nombreuses connexions électriques. Selon un plan parallèle au plan de détection P, l'enceinte de protection s'étend selon une épaisseur de 2 ou 3 mm autour de la matrice de pixels 22.

On a schématisé, par des flèches en pointillés, le parcours, dans l'échantillon 10, des photons diffusant dans ce dernier pour former le rayonnement rétrodiffusé 14. Le rayonnement rétrodiffusé 14 émane de la surface S de l'échantillon 10 selon différentes zones élémentaires de rétrodiffusion distinctes de la zone élémentaire d'illumination 18. On désigne par le terme "distance de rétrodiffusion" une distance séparant une zone élémentaire de rétrodiffusion de la zone élémentaire d'illumination 18. La figure 1B est une vue de dessus de la surface de l'échantillon. On a représenté, sur les figures 1A et 1B, deux zones élémentaires de rétrodiffusion $13_1$ et $13_2$ s'étendant respectivement à deux distances de rétrodiffusion $d_1$ et $d_2$ de la zone élémentaire d'illumination 18, et desquelles émanent un rayonnement de rétrodiffusé $14_1$ et un rayonnement rétrodiffusé $14_2$. Le terme zone élémentaire de rétrodiffusion désigne une zone de la surface de l'échantillon dont la dimension est telle que la zone peut être considérée comme étant située à une même distance de la zone élémentaire d'illumination 18. Une zone élémentaire de rétrodiffusion peut être ponctuelle. L'aire d'une zone de rétrodiffusion est alors inférieure à 1 mm$^2$, voire à 100 $\mu$m$^2$. Une zone élémentaire de rétrodiffusion peut également décrire tout ou partie d'un anneau, de faible épaisseur, de préférence inférieure à 1 mm, voire à 500 $\mu$m, voire 100 $\mu$m ou 50 $\mu$m s'étendant autour de la zone élémentaire d'illumination 18.

Comme mentionné dans EP3054281, plus la distance de rétrodiffusion est faible, plus le trajet, dans l'échantillon, des photons formant le rayonnement rétrodiffusé, s'étend faiblement en profondeur dans l'échantillon. Ainsi, plus la distance de rétrodiffusion est faible, plus le rayonnement rétrodiffusé est représentatif d'une partie superficielle de l'échantillon. A l'inverse, plus la distance de rétrodiffusion est importante, plus le rayonnement rétrodiffusé est représentatif d'une partie profonde de l'échantillon.

Par le biais du système optique 19, chaque pixel de la matrice de pixels 22 est optiquement couplé à une zone élémentaire de rétrodiffusion. On comprend que ce dispositif permet une détection de rayonnements rétrodiffusés selon de faibles distances de rétrodiffusion, typiquement de quelques dizaines de microns, ce qui permet une analyse correcte de la partie la plus superficielle de l'échantillon, s'étendant selon quelques dizaines de microns sous la surface S. Cependant, le dispositif est encombrant. Il n'est pas adapté à une utilisation nomade, en étant porté par une personne ou un animal.

Les inventeurs ont testé une configuration, nettement plus compacte, représentée sur la figure 1C. Elle comporte un capteur d'image 20, similaire au capteur décrit en lien avec la figure 1A. Sur le support du capteur d'image 21 est agencée une source de lumière ponctuelle 11, par exemple une diode électroluminescente. Le capteur d'image 20 est appliqué directement contre l'échantillon 10. Cependant, selon une telle configuration, la distance, parallèlement à la surface S de l'échantillon, entre le centre de la source de lumière 11 et la matrice de pixels 22 ne peut pas être inférieure à 3 ou 4 mmm. Cela est dû aux dimensions de l'enceinte de protection 23 s'étendant autour de la matrice de pixels 22. De ce fait, selon une telle configuration, la distance de rétrodiffusion ne peut pas être inférieure à une distance minimale $d_{min}$, ici égale à 4 mm, ce qui ne rend pas envisageable une détermination des propriétés optiques de diffusion et/ou d'absorption de la partie la plus superficielle de l'échantillon. De plus, même lorsque les propriétés optiques de l'échantillon sont homogènes, une telle distance s'avère trop importante pour caractériser correctement les propriétés de diffusion et/ou d'absorption de l'échantillon, comme expliqué ci-après.

Les inventeurs ont effectué des essais expérimentaux visant à comparer les performances des configurations respectivement représentées sur les figures 1A et 1C. Pour cela, ils sont utilisés des échantillons de calibration prenant la forme de fantômes dont les propriétés optiques de diffusion et d'absorption sont connues. Pour réaliser les essais, ils ont utilisé les composants suivants :

- Source de lumière 10 de la configuration "à distance" de la figure 1A : diode laser Power Technologies Inc de longueur d'onde $\lambda$ = 690 nm.
- Source de lumière 10 de la configuration "au contact" de la figure 1C : diode électroluminescente (Kingbright KPTD-1608SURK) émettant selon une bande spectrale centrée sur une longueur d'onde $\lambda$ = 640 nm et un angle d'émission d'environ 60°.
- Capteur d'image 20 : capteur CMOS UI-1492-LE IDS imaging.

Dans la configuration à distance, le système optique 19 est un objectif Xenoplan 1.4/23-0902, Schneider Kreuznach - 23 mm, disposé à une distance de 90 mm de la surface de l'échantillon. Une densité optique, générant une atténuation de $10^{-2}$, a été disposée entre le laser et le miroir 122. Deux filtres polarisants sont également disposés respectivement entre la source de lumière 11 et l'échantillon 10 ainsi qu'entre l'échantillon 10 et le capteur 20. La densité optique et les filtres polarisants sont optionnels et ne sont pas représentés sur la figure 1A. Les filtres polarisants permettent d'éviter que la réflexion diffuse du faisceau incident 12, à la surface de l'échantillon, parvienne au capteur d'image 20.

Différents échantillons de calibration ont été utilisés, présentant un même coefficient de diffusion réduit $\mu_s'$ et différents coefficients d'absorption $\mu_a$. Lors de chaque essai, on a déterminé les coefficients de diffusion et d'absorption selon la méthode décrite dans EP3054281, et plus particulièrement dans les paragraphes 87 à 95 de cette demande de brevet. D'une façon générale, on obtient les coefficients ($\mu_a$, $\mu_s'$) par une comparaison entre :

- une réflectance mesurée à différentes distances de rétrodiffusion ;
- une réflectance modélisée, à chaque distance de rétrodiffusion, en prenant en compte différents coefficients de diffusion réduit $\mu_s'$ et différents coefficient d'absorption $\mu_a$.

On détermine alors le couple ($\mu_a$, $\mu_s'$) qui correspond le mieux à la réflectance mesurée aux différentes distances de rétrodiffusion.

La réflectance diffuse correspond à une intensité d'un rayonnement de rétrodiffusion détecté normalisée par l'intensité du faisceau lumineux incident à l'échantillon, et pouvant être affectée de facteurs correctifs, prenant en compte la réponse des composants utilisés, par exemple le capteur d'image, les composants optiques ou la source de lumière.

La configuration à distance a tout d'abord été mise en œuvre, et on a déterminé le couple ($\mu_a$, $\mu_s'$) de six échantillons de calibration à partir du signal rétrodiffusé à différentes distances de rétrodiffusion, comprises entre 500 $\mu$m et 5 mm, selon un pas d'échantillonnage de 80 $\mu$m. Les résultats de cet essai sont représentés par un point sur les figures 2A et 2B.

La figure 2A représente l'erreur relative $\varepsilon$ affectant la détermination du coefficient d'absorption $\mu_a$ (axe des ordonnées) en fonction du coefficient d'absorption réel $\mu_a$ de chaque échantillon (axe des abscisses). La figure 2B représente l'erreur relative $\varepsilon'$ affectant la détermination du coefficient de diffusion réduit $\mu_s'$ (axe des ordonnées) en fonction du coefficient d'absorption réel $\mu_a$ de chaque échantillon (axe des abscisses).

La configuration au contact a été mise en oeuvre, en se basant sur un a priori concernant la valeur du coefficient de diffusion réduit $\mu_s$'. On a ainsi déterminé les coefficients d'absorption $\mu_a$ de chaque échantillon de calibration. Les résultats de cet essai sont représentés par un triangle sur la figure 2A.

La configuration au contact a été mise en œuvre, sans a priori concernant la valeur du coefficient de diffusion réduit $\mu_s$'. On a ainsi déterminé les coefficients d'absorption et de diffusion réduit ($\mu_a$, $\mu_s$') de chaque échantillon de calibration. Les résultats de cet essai sont représentés par un cercle sur les figures 2A et 2B.

Les résultats présentés sur les figures 2A et 2B mènent aux conclusions suivantes :

- la configuration à distance présente des performances optimales et peut être considérée comme une méthode de référence ;
- la configuration au contact, avec un a priori quant au coefficient de diffusion réduit, permet une estimation acceptable du coefficient d'absorption (cf. figure 2A), avec une plage d'erreur inférieure à ou de l'ordre de $\pm$ 5% ;
- la configuration au contact, sans a priori quant au coefficient de diffusion réduit, peut entraîner une erreur de mesure importante, pouvant atteindre $\pm$ 15%, affectant la détermination des coefficients d'absorption (figure 2A) et de diffusion réduit (figure 2B). De plus, les erreurs affectant respectivement ces deux coefficients semblent corrélées en valeur absolue.

Les inventeurs ont attribué la moindre performance de la configuration au contact au fait que lorsque la distance minimale de rétrodiffusion ($d_{min}$) est faible, typiquement inférieure à 1 mm, la réflectance varie essentiellement en fonction du coefficient de diffusion réduit. Les figures 2C et 2D illustrent ce constat. Sur ces figures, on a représenté respectivement l'évolution de la réflectance (axe des ordonnées) en fonction de la distance de rétrodiffusion (axe des abscisses), en considérant respectivement différents coefficients de diffusion réduits $\mu_s$' et différents coeffcients d'absorption $\mu_a$. Ces valeurs de réflectance ont été obtenues par modélisation de l'équation de diffusion. Sur ces deux figures, les flèches symbolisent des valeurs croissantes des coefficients $\mu_a$ (sur la figure 2D) et $\mu_s$' (sur la figure 2C).

La figure 2C a été obtenue en faisant varier $\mu_s$' de 10 cm$^{-1}$ à 80 cm$^{-1}$ par pas de 4 cm$^{-1}$, le coefficient d'absorption $\mu_a$ de l'échantillon modélisé étant fixé à 1 cm$^{-1}$. L'indice de réfraction de l'échantillon est fixé à 1.33, l'échantillon étant disposé dans l'air, d'indice de réfraction égal à 1. Le coefficient d'anisotropie g est fixé à 0.8. Le coefficient d'anisotropie, connu de l'homme du métier, relie le coefficient de diffusion réduit $\mu_s$' au coefficient de diffusion $\mu_s$ selon l'expression

$$\mu'_s = \mu_s \, (1 - g)$$

La figure 2D a été obtenue en faisant varier $\mu_a$ de 0.01 cm$^{-1}$ à 4 cm$^{-1}$ par pas de 0.2 cm$^{-1}$, le coefficient d'absorption $\mu_s$' de l'échantillon modélisé étant fixé à 20 cm$^{-1}$. L'indice de réfraction de l'échantillon est fixé à 1.33, l'échantillon étant disposé dans l'air, d'indice de réfraction égal à 1. Le coefficient d'anisotropie g est fixé à 0.8.

On observe que lorsque la distance de rétrodiffusion est inférieure à 1 mm, la réflectance n'évolue presque pas en fonction de $\mu_a$. A l'inverse, à de faibles distances de rétrodiffusion, la réflectance évolue en fonction de $\mu_s$'. Au-delà d'une distance de rétrodiffusion de 1 mm, l'intensité du rayonnement rétrodiffusée décroît à la fois en fonction de $\mu_a$ et de $\mu_s$'. En prenant en compte une telle évolution, il est donc difficile d'estimer à la fois le coefficient d'absorption $\mu_a$ et le coefficient de diffusion réduit $\mu_s$'. En effet, en l'absence de mesure à faible distance, c'est-à-dire à une distance de rétrodiffusion inférieure à 1 mm, l'estimation simultanée des coefficients d'absorption $\mu_a$ et de diffusion réduit $\mu_s$' est délicate, car la réflectance, c'est-à-dire l'intensité du rayonnement rétrodiffusé normalisée, est influencée de la même manière par ces deux coefficients.

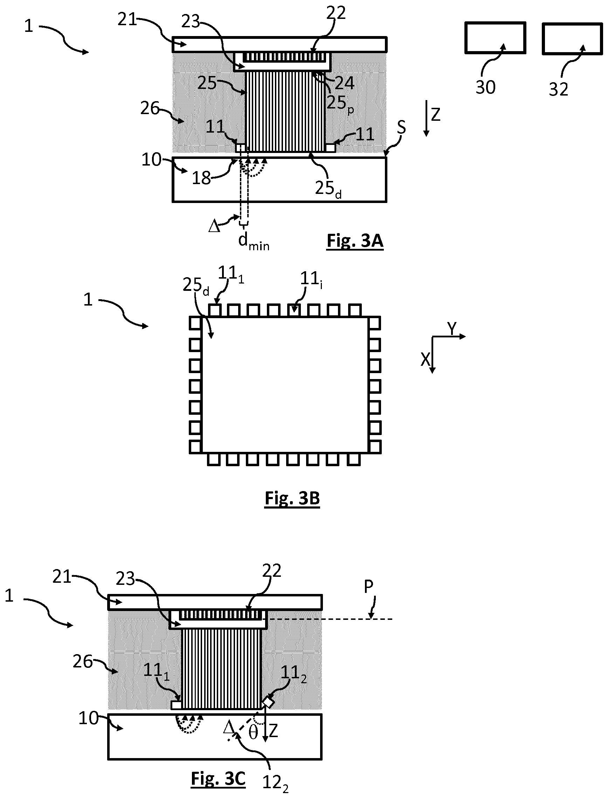

Ayant constaté ce qui précède, afin de pouvoir réaliser des estimations satisfaisantes des propriétés d'absorption et de diffusion en utilisant un dispositif compact et portable, les inventeurs ont conçu un dispositif tel que celui représenté sur la figure 3A. Le dispositif comporte un capteur d'image 20 tel que précédemment décrit. Il comporte une source de lumière 11, apte à générer un faisceau d'illumination 12 se propageant vers l'échantillon. L'intersection du faisceau d'illumination 12 avec la surface S de l'échantillon forme une zone élémentaire d'illumination 18. La zone élémentaire d'illumination peut être ponctuelle, c'est-à-dire présentant une aire inférieure à 5 mm$^2$, de préférence inférieure à 2 mm$^2$ ou 1 mm$^2$. Elle peut également former un motif dont une dimension est inférieure à 5 mm, voire 2 mm ou 1 mm. Il peut par exemple s'agir d'une ligne fine. Sur la figure 3A, le faisceau d'illumination 12 n'a pas été représenté car le dispositif 1 est appliqué contre l'échantillon 10.

La source de lumière 11 peut par exemple émettre le faisceau d'illumination 12 dans une bande spectrale d'émission $\Delta\lambda$ s'étendant dans le visible ou le proche infra-rouge, soit comprise entre 400 nm et 950 nm. De préférence, la largeur de bande est inférieure à 100 nm, voire inférieure à 50 nm ou à 20 nm. Par largeur de bande, on entend la largeur à mi-hauteur de la bande spectrale d'émission.

Dans l'exemple représenté sur la figure 3A, la source de lumière 11 est une diode électroluminescente telle que décrit en lien avec les précédents exemples. De façon alternative, la source de lumière peut être une diode laser de faible puissance. Il peut également s'agir d'une extrémité d'une fibre optique, la fibre optique étant couplée à une source de lumière.

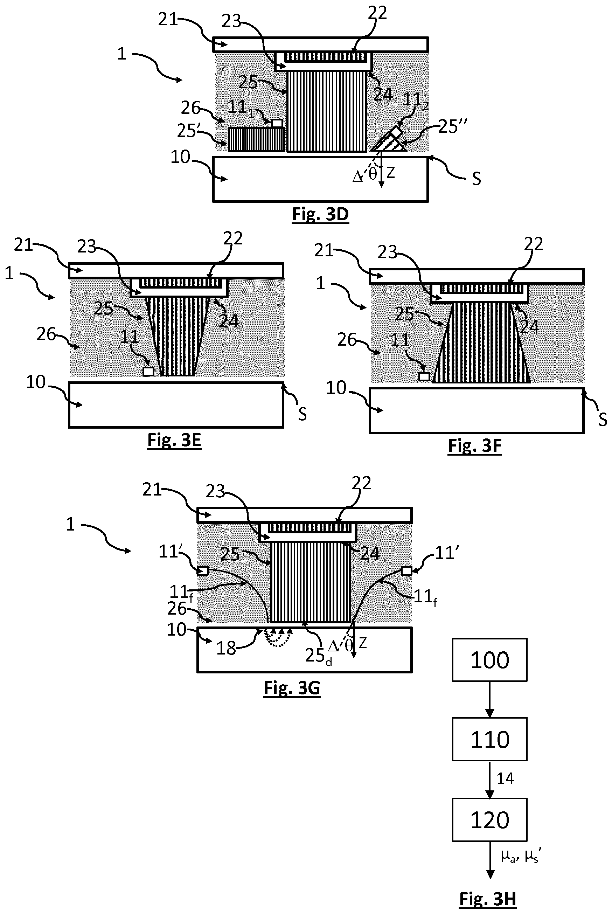

Le dispositif selon l'invention comporte un faisceau de fibres optiques formant une plaque de fibres optiques, usuellement désigné par le terme anglosaxon "fiber optic plate". Le faisceau de fibres optiques s'étend entre une surface proximale $25_p$, disposée contre le capteur d'image 20 et une surface distale $25_d$, destinée à être appliquée contre l'échantillon 10 à analyser. Le faisceau de fibres optiques 25 comporte une pluralité de fibres optiques assemblées les unes aux autres. Les fibres optiques sont accolées les unes aux autres. Le faisceau de fibres optiques peut comporter plusieurs centaines, voire milliers de fibres optiques. Il permet de transporter une image du rayonnement rétrodiffusé 14 émanant de la surface S de l'échantillon vers la matrice de pixels 22 du capteur d'image 20. Selon l'invention, les fibres optiques du faisceau 25 s'étendent selon un axe Z, dit axe d'extension, de telle sorte que le faisceau 25 permet de transporter l'image formée à la surface du tissu, selon l'axe d'extension, vers la matrice de pixels 22. Le faisceau de fibre optique est de préférence assemblé au capteur d'image. Les fibres optiques du faisceau s'étendent parallèlement à l'axe d'extension Z, comme décrit en lien avec les figures 3A, 3C, 3D et 3G, ou peuvent être inclinées par rapport à ce dernier, comme décrit en lien avec les figures 3E et 3F. Lorsqu'elles sont inclinées par rapport à l'axe d'extension, elles peuvent former un faisceau convergent ou divergent à partir du capteur d'image 20. La surface proximale $25_p$ du faisceau s'étend parallèlement au plan de détection P, tandis que la surface distale $25_d$ du faisceau s'étend parallèlement à la surface S de l'échantillon. De préférence, le plan de détection est parallèle à la surface de l'échantillon. C'est en particulier le cas lorsque l'échantillon examiné est déformable, lorsqu'en appliquant le dispositif contre l'échantillon, la surface de l'échantillon S s'étend parallèlement à la surface distale $25_d$ du faisceau. Dans cet exemple, la surface proximale $25_p$ est collée sur la fenêtre transparente 24. Dans cet exemple, l'axe d'extension Z du faisceau 25 est orthogonal au plan de détection P, ce qui correspond à une configuration préférée.

Par le recours du faisceau 25, dans tout ou partie de la matrice de pixels 22, les fibres optiques du faisceau de fibres débouchent sur des pixels adjacents les uns des autres.

De préférence, l'aire de la surface proximale $25_p$ est inférieure ou égale à l'aire de la matrice de pixels 22. Ainsi, dans un plan transversal XY selon lequel s'étendent les surfaces proximale et distale, le faisceau 25 est inscrit dans le périmètre défini par la matrice de pixels. Cela permet de disposer la source de lumière 11 contre le faisceau 25, de façon à minimiser la distance $d_{min}$, parallèlement à la surface S de l'échantillon (ou dans le plan de détection P), entre la source de lumière 11 et la matrice de pixels 22. Ainsi, le dispositif permet de détecter un signal rétrodiffusé émanant de la surface de l'échantillon S selon une distance de rétrodiffusion minimale $d_{min}$ inférieure à celle de la configuration décrite en lien avec la figure 1C. Cette distance correspond à la distance la plus courte, parallèlement au plan de l'échantillon, entre la source de lumière 11 et le faisceau de fibre optiques 25. Selon l'invention, la distance $d_{min}$ est inférieure à 1 mm, et encore de préférence inférieure à 500 $\mu$m. Elle correspond à une distance, parallèlement au plan de détection, entre l'axe d'incidence $\Delta$ du faisceau produit par la source de lumière et la matrice de pixels 22. Le recours au faisceau 25 de fibre optique, dont l'aire est inférieure ou égale à l'aire de la matrice de pixels 22, permet de réduire significativement la distance, parallèlement à la surface de l'échantillon, ou selon le plan de détection P, entre la source de lumière 11 et les pixels du capteur d'image 20.

On note que le recours à un faisceau 25 de fibres optiques permet d'effectuer une analyse du rayonnement rétrodiffusé d'un échantillon à l'aide d'un capteur d'image 20 standard, sans nécessiter une modification de ce dernier.

En fonction de la résolution spatiale que l'on souhaite obtenir, le diamètre de chaque fibre du faisceau peut être compris entre 5 $\mu$m et 100 $\mu$m ou 200 $\mu$m, par exemple 70 $\mu$m. Afin de réduire des fuites de lumière entre des fibres adjacentes, des fibres du faisceau peuvent être avantageusement gainées par un matériau opaque.

La source de lumière 11 peut affleurer la surface distale $25_d$ du faisceau de fibres 25, ou être disposée en retrait par rapport à cette dernière, par exemple selon un recul inférieur à 1 cm, voire à 5 mm. Le fait de rapprocher la source de lumière 11 de la surface distale 25 permet de limiter l'aire de la zone élémentaire d'illumination 18, du fait de la divergence du faisceau lumineux incident 12.

La surface distale $25_d$ du faisceau 25 peut être enduite d'un matériau biocompatible transparent, facilitant une utilisation du dispositif sur la peau d'un être humain ou d'un animal.

Les inventeurs ont utilisé un faisceau de fibre optiques de diamètre 70 $\mu$m, d'ouverture numérique 0.84, fourni par Paradigm Otptics référence PA0470 de diamètre 1 pouce selon le plan XY et d'épaisseur ½ pouce selon l'axe Z. Cela a permis d'obtenir une distance minimale de rétrodiffusion $d_{min}$ égale à 470 $\mu$m.

Le capteur d'image 20, la source de lumière 11 et le faisceau 25 peuvent être solidaires d'un support de maintien 26, ce dernier pouvant être un matériau d'encapsulation en plastique rigide.

Selon les exemples représentés sur les figures 3A, 3B et 3C, le dispositif comporte plusieurs sources de lumière 11. On a représenté, sur la figure 3B, différentes sources de lumières $11_1...11_i...$agencées autour d'un faisceau de fibres optiques 25, à une distance aussi faible que possible de ce dernier, cette distance étant inférieure à 1 mm. On peut alors utiliser des sources de lumière émettant selon des bandes spectrales $\Delta\lambda$ différentes les unes des autres. Cela permet une caractérisation des propriétés optiques de l'objet selon les différentes bandes spectrales utilisées. De préférence, la largeur des bandes spectrales de chaque source de lumière est inférieure à 100 nm ou à 50 nm ou à 20 nm. Par largeur de bande, on entend la largeur à mi-hauteur du pic d'émission correspondant à la bande spectrale.

Sur les figures 3A et 3C, les fibres optiques sont parallèles à l'axe d'extension Z.

Chaque source de lumière est de préférence incluse dans le support de maintien, de telle sorte que le dispositif forme une pièce monobloc.

Selon un mode de réalisation, représenté sur la figure 3C, le dispositif comporte une source de lumière 11 émettant un faisceau incident 12 se propageant selon un axe d'incidence Δ incliné par rapport à l'axe d'extension Z selon lequel s'étend le faisceau incident 25. L'angle d'inclinaison θ entre l'axe d'incidence Δ et l'axe d'extension Z peut par exemple être compris entre 10° et 70° à 80°. Il est de préférence compris entre 30° et 45°. Cela permet de rapprocher, dans le plan défini par la surface de l'échantillon, la zone élémentaire d'illumination 18 de la matrice de pixels 22. L'angle d'inclinaison peut par exemple être de 35°.

Selon un mode de réalisation, représenté sur la figure 3D, le dispositif comporte une première source de lumière $11_1$ optiquement couplée à un faisceau auxiliaire 25' de fibres optiques 25', s'étendant entre la première source de lumière $11_1$ et une surface du support de maintien 26 destinée à être appliquée face à la surface de l'échantillon 10. De façon optionnelle, le dispositif comporte une deuxième source de lumière $11_2$ optiquement couplée à un deuxième faisceau auxiliaire 25" de fibres optiques auxiliaire. Ce dernier est configuré pour diriger la lumière générée par la deuxième source de lumière $11_2$ selon un axe d'incidence Δ formant un angle d'inclinaison θ avec l'axe d'extension Z. Comme décrit en lien avec la figure 3C, l'angle d'inclinaison peut être compris entre 10° et 70° ou 80°. Il est de préférence compris entre 30° et 45°.

Selon des modes de réalisation représentés sur les figures 3E et 3F, le faisceau de fibres optiques 25 ne comporte pas des fibres étant toutes orientées parallèlement à l'axe d'extension Z. Ces fibres optiques forment un faisceau convergeant autour de l'axe d'extension Z. Sur la figure 3E, le faisceau 25 converge vers la surface de l'échantillon. Cela permet de rapprocher les distances de rétrodiffusion les unes des autres. Sur la figure 3F, le faisceau 25 diverge à partir du capteur d'image, ce qui permet de gagner en sensibilité, puisque plusieurs fibres peuvent ainsi être couplées à un même pixel du capteur d'image.

Selon un mode de réalisation, représenté sur la figure 3G, au moins une source de lumière est fibrée, dans le sens ou une fibre optique d'illumination $11_f$ s'étend à partir de la source de lumière, vers l'échantillon. On a représenté une source de lumière 11', incluse dans le support de maintien 26, et à partir de laquelle s'étend une fibre d'illumination $11_f$ jusqu'à une surface du support de maintien apte à être appliquée en vis-à-vis de l'échantillon 10. On a également représenté une autre source lumineuse 11', incluse dans le support de maintien 26, et à partir de laquelle s'étend une fibre d'illumination $11_f$ jusqu'à une surface du support de maintien apte à être appliquée en vis-à-vis de l'échantillon 10, de façon à propager un faisceau d'illumination selon un axe d'incidence Δ formant un angle d'inclinaison θ par rapport à l'axe d'extension Z. L'angle d'inclinaison est similaire à celui décrit en lien avec la figure 3C ou 3D. L'extrémité de la fibre d'illumination $11_f$ forme alors la source de lumière 11. L'utilisation d'une fibre permet de rapprocher la source de lumière du faisceau de fibres optiques 25.

Le dispositif peut être relié à un processeur 30, comportant une mémoire 32, dans laquelle sont stockées des instructions pour pouvoir permettre une détermination des propriétés optique d'absorption et/ou de diffusion à partir des rayonnements rétrodiffusés détectés, selon différentes distances de rétrodiffusion, par le capteur d'image 20. Le procédé peut comporter les étapes suivantes, illustrées sur la figure 3H :

Etape 100 : application du dispositif contre l'échantillon, au contact de ce dernier. De préférence, après l'application, la surface S de l'échantillon s'étend parallèlement à la surface distale $25_d$ du faisceau 25.

Etape 110 : illumination de l'échantillon par la source de lumière ou les sources de lumière et détection d'un rayonnement rétrodiffusé 14 par l'échantillon selon au moins deux distances de rétrodiffusion différentes et non nulles.

Etape 120 : à partir du rayonnement rétrodiffusé par le capteur d'image, estimation, par le processeur 30, de propriétés optiques de diffusion et/ou d'absorption, en se basant sur des algorithmes. Les algorithmes mis en œuvre sont par exemple basés sur une comparaison entre des valeurs d'intensité du rayonnement rétrodiffusé mesurées et des valeurs modélisées en considérant différentes valeurs de propriétés optiques, comme décrit dans EP3054281. On tire alors profit qu'au moins une distance de rétrodiffusion considérée est inférieure à 1 mm, voire à 500 μm, ce qui permet une meilleure estimation des propriétés optiques.

Le dispositif représenté sur la figure 3A a été utilisé en utilisant une seule source de lumière, correspondant à la diode électroluminescente décrite en lien avec les essais précédents. On a mis en œuvre le dispositif sur des échantillons de calibration, de façon similaire aux essais décrits en relation avec les figures 2A et 2B.

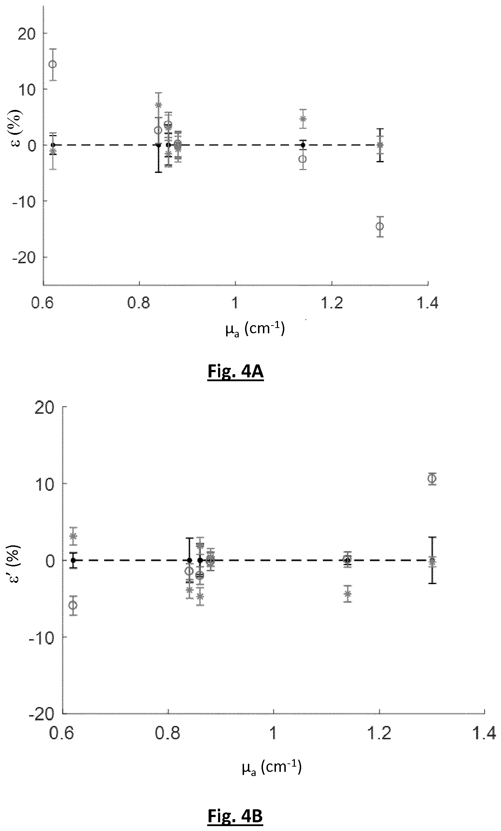

La figure 4A représente l'erreur relative ε affectant la détermination du coefficient d'absorption $\mu_a$ (axe des ordonnées) en fonction du coefficient d'absorption réel $\mu_a$ de chaque échantillon (axe des abscisses). Cette figure montre :

- les résultats mettant en œuvre la configuration à distance telle que représentée sur la figure 1A, ces résultats correspondant aux marques en forme de points sombres ;

- les résultats mettant en œuvre la configuration au contact telle que représentée sur la figure 1C ces résultats correspondant aux marques en forme de cercle ;
- les résultats mettant en œuvre la configuration selon l'invention, telle que représentée sur la figure 3A ces résultats correspondant aux marques en forme d'étoile ;

La figure 4B représente l'erreur relative $\varepsilon'$ affectant la détermination du coefficient de diffusion réduit $\mu_s'$ (axe des ordonnées) en fonction du coefficient d'absorption réel $\mu_a$ de chaque échantillon (axe des abscisses). Les marques représentées sur la figure 4B correspondent à celles de la figure 4A.

On observe que le recours au faisceau de fibres optiques 25 permet une amélioration significative des performances de la configuration au contact, l'erreur relative associée à la détermination des coefficients de diffusion ou d'absorption étant inférieure à $\pm 5\%$. On obtient ainsi des performances proches de celles offertes par la configuration à distance.

L'invention pourra être mise en œuvre pour étudier les propriétés optiques d'absorption ou de diffusion d'objets, et en particuliers des tissus biologiques, par exemple la peau d'un être humain ou d'un animal. Les propriétés optiques d'absorption ou de diffusion peuvent permettre de quantifier la présence d'éléments chromophores dans les tissus, ou d'effectuer des mesures relatives à l'oxymétrie, cela à des fins de diagnostic ou de suivi de traitement d'individus. L'invention peut avoir des applications dans d'autres domaines industriels, par exemple l'agroalimentaire.

## Revendications

1. Dispositif de mesure d'un rayonnement (14) rétrodiffusé par un échantillon (10) comportant :

    - au moins une source de lumière (11) apte à émettre un faisceau lumineux (12), selon un axe d'incidence ($\Delta$), vers une surface de l'échantillon (S), de façon à former, sur ladite surface, une zone élémentaire d'illumination (18) ;
    - un capteur d'image (20), pour former une image du rayonnement rétrodiffusé par l'échantillon lorsque ce dernier est illuminé par la source de lumière, le capteur d'image s'étendant selon un plan de détection (P) ; et
    - un faisceau de fibres optiques (25), formant une plaque s'étendant, selon un axe d'extension (Z), entre une surface proximale ($25_p$) et une surface distale ($25_d$), la surface proximale étant appliquée contre le capteur d'image, la surface distale du faisceau de fibres étant configurée pour être appliquée au contact de la surface de l'échantillon, la surface distale étant configurée pour s'étendre parallèlement à la surface de l'échantillon;

le dispositif étant tel que la source de lumière (11) est disposée autour du faisceau de fibres optiques (25), à une distance inférieure à 1 mm du faisceau de fibres optiques, la distance étant déterminée parallèlement à la surface distale.

2. Dispositif selon l'une quelconque des revendications précédentes, dans lequel la source de lumière (11) est disposée autour du faisceau de fibres optique (25), entre le capteur d'image (20) et l'échantillon (10).

3. Dispositif selon l'une quelconque des revendications précédentes, dans lequel la source de lumière (11) affleure la surface distale.

4. Dispositif selon l'une quelconque des revendications précédentes, dans lequel l'axe d'extension (Z) est perpendiculaire au plan de détection (P).

5. Dispositif selon l'une quelconque des revendications précédentes, dans lequel les fibres optiques du faisceau (25) de fibres optiques :

    - sont orientées parallèlement à l'axe d'extension (Z) ;
    - ou forment un faisceau divergent ou convergent à partir du capteur d'image (20).

6. Dispositif selon l'une quelconque des revendications précédentes, comportant plusieurs sources de lumière ($11_1...11_i$), chaque source de lumière étant disposée autour du faisceau de fibres optiques.

7. Dispositif selon la revendication 6, dans lequel au moins deux sources de lumière (10, $10_i$) émettent des faisceaux lumineux respectivement selon des bandes spectrales différentes.

8. Dispositif selon l'une quelconque des revendications précédentes, dans lequel au moins une source de lumière est telle que l'axe d'incidence ($\Delta$) du faisceau lumineux produit par cette dernière est sécant par de l'axe d'extension (Z) du faisceau (25) de fibres optiques.

9. Dispositif selon l'une quelconque des revendications précédentes, dans lequel au moins une source de lumière (11) est :

    - une diode électroluminescente ;
    - ou une diode laser ;
    - ou une extrémité d'une fibre optique ($11_f$), dite fibre optique d'illumination, la fibre optique d'illumination étant couplée à une source lumineuse (11').

10. Dispositif selon l'une quelconque des revendications précédentes, dans lequel le capteur d'image, le faisceau de fibres optiques (25) et chaque source de lumière sont maintenus solidaires les uns des autres par un support de maintien (26).

11. Dispositif selon l'une quelconque des revendications précédentes, dans lequel le capteur d'image (20) comporte une matrice de pixels (22), de telle sorte que la distance, parallèlement à la surface distale, entre au moins une source de lumière et la matrice de pixels est inférieure à 500 $\mu$m.

12. Procédé d'estimation d'une propriété optique de diffusion et/ou d'absorption d'un échantillon, à l'aide d'un dispositif selon l'une quelconque des revendications précédentes, le procédé comportant les étapes suivantes :

a) illumination de l'échantillon à l'aide de la source de lumière du dispositif, de façon à former, sur une surface de l'échantillon, une zone élémentaire d'illumination ;
b) formation, à l'aide du capteur d'image, d'une image représentant un rayonnement rétrodiffusé par l'échantillon sous l'effet de l'illumination, le rayonnement rétrodiffusé émanant de la surface de l'échantillon, à l'extérieur de la zone élémentaire d'illumination, à plusieurs distances, dites distances de rétrodiffusion, de la zone élémentaire d'illumination ;
c) détermination d'une propriété optique de diffusion et/ou d'absorption en fonction d'une intensité du rayonnement rétrodiffusé, à différentes distances de rétrodiffusion, au moins une distance de rétrodiffusion étant inférieure à 1 mm ou à 500 $\mu$m.

**Patentansprüche**

1. Vorrichtung zur Messung einer Rückstreustrahlung (14) von einer Probe (10), die umfasst:

- mindestens eine Lichtquelle (11), die dazu geeignet ist, einen Lichtstrahl (12) in einer Einfallachse ($\Delta$) auf eine Oberfläche der Probe (S) zu emittieren, so dass auf der Oberfläche eine elementare Beleuchtungszone (18) gebildet wird;
- einen Bildsensor (20) zum Bilden eines Bildes der Rückstreustrahlung von der Probe, wenn Letztere von der Lichtquelle beleuchtet wird, wobei sich der Bildsensor in einer Erkennungsebene (P) erstreckt;
und
- ein Lichtwellenleiterbündel (25), das eine Platte bildet und sich in einer Erstreckungsachse (Z) zwischen einer proximalen Oberfläche (25$_p$) und einer distalen Oberfläche (25$_d$) erstreckt, wobei die proximale Oberfläche am Bildsensor angelegt ist, wobei die distale Oberfläche des Lichtwellenleiterbündels dazu konfiguriert ist, in Kontakt mit Probenoberfläche angelegt zu werden, wobei die distale Oberfläche dazu konfiguriert ist, sich parallel zur Probenoberfläche zu erstrecken;

wobei die Vorrichtung so beschaffen ist, dass die Lichtquelle (11) um das Lichtwellenleiterbündel (25) in einem Abstand von weniger als 1 mm vom Lichtwellenleiterbündel angeordnet ist, wobei der Abstand parallel zur distalen Oberfläche bestimmt ist.

2. Vorrichtung nach einem der vorhergehenden Ansprüche, wobei die Lichtquelle (11) um das Lichtwellenleiterbündel (25) zwischen dem Bildsensor (20) und der Probe (10) angeordnet ist.

3. Vorrichtung nach einem der vorhergehenden Ansprüche, wobei die Lichtquelle (11) die distale Oberfläche leicht berührt.

4. Vorrichtung nach einem der vorhergehenden Ansprüche, wobei die Erstreckungsachse (Z) senkrecht zur Detektionsebene (P) verläuft.

5. Vorrichtung nach einem der vorhergehenden Ansprüche, wobei die Lichtwellenleiter des Lichtwellenleiterbündels (25):

- parallel zur Erstreckungsachse (Z) ausgerichtet sind;
- oder ein vom Bildsensor (20) ausgehendes divergierendes oder konvergierendes Bündel bilden.

6. Vorrichtung nach einem der vorhergehenden Ansprüche, die mehrere Lichtquellen (11$_1$...11$_i$) umfasst, wobei jede Lichtquelle um das Lichtwellenleiterbündel angeordnet ist.

7. Vorrichtung nach Anspruch 6, wobei mindestens zwei Lichtquellen (10, 10$_i$) jeweils Lichtstrahlen unterschiedlicher Spektralbänder emittieren.

8. Vorrichtung nach einem der vorhergehenden Ansprüche, wobei mindestens eine Lichtquelle so beschaffen ist, dass die Einfallsachse ($\Delta$) des Lichtstrahls, der von Letzterer erzeugt wird, sekantenartig in Bezug auf die Erstreckungsachse (Z) des Lichtwellenleiterbündes (25) verläuft.

9. Vorrichtung nach einem der vorhergehenden Ansprüche, wobei mindestens eine Lichtquelle (11) Folgendes ist:

- eine Leuchtdiode;
- eine Laserdiode;
- oder ein Ende eines Lichtwellenleiters (11$_f$), Beleuchtungslichtwellenleiter genannt, wobei der Beleuchtungslichtwellenleiter mit einer Lichtquelle (11') verbunden ist.

10. Vorrichtung nach einem der vorhergehenden Ansprüche, wobei der Bildsensor, das Lichtwellenleiterbündel (25) und jede Lichtquelle durch einen Halteträger (26) miteinander festgehalten werden.

11. Vorrichtung nach einem der vorhergehenden Ansprüche, wobei der Bildsensor (20) eine Pixelmatrix (22) umfasst, so dass der Abstand parallel zur distalen Oberfläche zwischen mindestens einer Lichtquelle und der Pixelmatrix kleiner als 500 μm ist.

12. Verfahren zur Bewertung einer optischen Diffusions- und/oder Absorptionseigenschaft einer Probe mithilfe einer Vorrichtung nach einem der vorhergehenden Ansprüche, wobei das Verfahren die folgenden Schritte umfasst:

a) Beleuchtung der Probe mithilfe der Lichtquelle der Vorrichtung, so dass auf der Oberfläche der Probe eine elementare Beleuchtungszone gebildet wird;
b) Bildung eines Bildes, das eine Rückstreustrahlung von der Probe darstellt, mithilfe des Bildsensors unter der Wirkung der Beleuchtung, wobei die Rückstreustrahlung von der Oberfläche der Probe ausgeht, außerhalb der elementaren Beleuchtungszone in mehreren Abständen, Rückstreustrahlungsabstände genannt, von der elementaren Beleuchtungszone;
c) Bestimmung einer optischen Diffusions- und/oder Absorptionseigenschaft in Abhängigkeit von einer Stärke der Rückstreustrahlung in verschiedenen Rückstreustrahlungsabständen, wobei mindestens ein Rückstreustrahlungsabstand kleiner als 1 mm oder 500 μm ist.

**Claims**

1. Device for measuring radiation (14) backscattered by a sample (10) including:

- at least one light source (11) that is configured to emit a light beam (12), along an axis of incidence (Δ), towards a surface of the sample (S) so as to form, on said surface, an elementary illumination zone (18);
- an image sensor (20) for forming an image of the radiation backscattered by the sample when the latter is illuminated by the light source, the image sensor lying in a detection plane (P); and
- a bundle of optical fibres (25) form a plate extending, along an axis of extension (Z), between a proximal surface (25$_p$) and a distal surface (25$_d$), the proximal surface being applied against the image sensor, the distal surface of the bundle of fibres being configured to be applied in contact with the surface of the sample, the distal surface being configured to extend parallel to the surface of the sample;

the device being such that the light source (11) is arranged around the bundle of optical fibres (25) at a distance of less than 1 mm away from the bundle of optical fibres, the distance being determined parallel to the distal surface.

2. Device according to any one of the preceding claims, wherein the light source (11) is arranged around the bundle of optical fibres (25), between the image sensor (20) and the sample (10).

3. Device according to any one of the preceding claims, wherein the light source (11) is flush with the distal surface.

4. Device according to any one of the preceding claims, wherein the axis of extension (Z) is perpendicular to the detection plane (P).

5. Device according to any one of the preceding claims, wherein the optical fibres of the bundle (25) of optical fibres:

- are oriented in parallel to the axis of extension (Z);
- or form a bundle diverging or converging away from/toward the image sensor (20).

6. Device according to any one of the preceding claims, including a plurality of light sources (11$_1$...11$_i$), each light source being arranged around the bundle of optical fibres.

7. Device according to Claim 6, wherein at least two light sources (10, 10$_i$) emit light beams in different spectral bands, respectively.

8. Device according to any one of the preceding claims, wherein at least one light source is such that the axis of incidence (Δ) of the light beam produced by the latter intersects the axis of extension (Z) of the bundle (25) of optical fibres.

9. Device according to any one of the preceding claims, wherein at least one light source (11) is:

- a light-emitting diode;
- or a laser diode;
- or an end of an optical fibre ($11_f$), referred to as the illuminating optical fibre, the illuminating optical fibre being coupled to a light source (11').

10. Device according to any one of the preceding claims, wherein the image sensor, the bundle of optical fibres (25) and each light source are held securely to one another by means of a holding support (26).

11. Device according to any one of the preceding claims, wherein the image sensor (20) includes a matrix-array of pixels (22), such that the distance, parallel to the distal surface, between at least one light source and the matrix-array of pixels is less than 500 $\mu$m.

12. Method for estimating a scattering and/or absorption optical property of a sample using a device according to any one of the preceding claims, the method including the following steps:

> a) illuminating the sample using the light source of the device so as to form, on a surface of the sample, an elementary illumination zone;
> b) forming, using the image sensor, an image representing radiation backscattered by the sample under the effect of the illumination, the backscattered radiation emanating from the surface of the sample outside of the elementary illumination zone at several distances, referred to as backscattering distances, away from the elementary illumination zone;
> c) determining a scattering and/or absorption optical property according to an intensity of the backscattered radiation, at various backscattering distances, at least one backscattering distance being less than 1 mm or than 500 $\mu$m.

**Fig. 1A**

**Fig. 1B**

**Fig. 1C**

**Fig. 2A**

**Fig. 2B**

**Fig. 2C**

**Fig. 2D**

Fig. 3A

Fig. 3B

Fig. 3C

**Fig. 3D**

**Fig. 3E**

**Fig. 3F**

**Fig. 3G**

**Fig. 3H**

**Fig. 4A**

**Fig. 4B**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2762064 A
- US 805532 A
- WO 2016181077 A1

- US 2016235303 A1
- EP 3054281 A

**Littérature non-brevet citée dans la description**

- **HAAHR R.** An Electronic Patch for Wearable Health Monitoring by Reflectance Pulse Oxymetry. *IEEE Transactions on biomedical circuits and systems,* 2011